# EUROPEAN PATENT SPECIFICATION

(11) **EP 3 851 851 B1**
(45) Date of publication and mention of the grant of the patent: **16.04.2025**
(21) Application number: 20151744.8
(22) Date of filing: 14.01.2020
(51) Int. Cl.: G01N 33/38

(54) **METHOD FOR MEASURING TEMPERATURE AND/OR HUMIDITY OF CEMENT, CONCRETE OR THE LIKE**
VERFAHREN ZUR MESSUNG EINER TEMPERATUR UND/ODER FEUCHTIGKEIT VON ZEMENT, BETON ODER DERGLEICHEN
PROCÉDÉ POUR MESURER LA TEMPÉRATURE ET/OU L'HUMIDITÉ DU CIMENT, DU BÉTON OU SIMILAIRE

(43) Date of publication of application: 21.07.2021
(73) Proprietor: SUPSI (Scuola Universitaria Professionale Della Svizzera Italiana), 6928 Manno (CH)
(72) Inventor: Giussani, Luca, 22070 Senna Comasco (CO) (IT); PORETTI, Giacomo, CH-6943 Vezia (CH); KNÜSEL, Alfons, CH-6275 Ballwil (CH); MONLEONE, Ricardo, CH-6863 Besazio (CH)
(74) Representative: M. Zardi & Co S.A.

(56) References cited:
- EP-A1- 2 919 007
- WO-A1-2013/030430
- DE-A1- 102011 056 548
- DE-A1- 102012 208 050
- FINLAND OY SAINT-GOBAIN: "P22 Accessories for flooring: Moisture sensor for floor screeds", 19 December 2019 (2019-12-19), pages 1 - 2, XP055710036, Retrieved from the Internet <URL:https://www.fi.weber/files/fi/2019-04/weberfloor-Moisture-Sensor-for-floor-screeds-Product-Datasheet.pdf> [retrieved on 20200630]

## Description

### Field of application:

The present invention relates to a method of measuring the temperature and/or humidity of cement, concrete or the like.

### Prior art

Sensor devices for measuring a temperature and/or humidity underneath the floor are known.

For instance, WO2006058350 discloses a device for detecting environmental parameters and includes at least one sensor for measuring the temperature under the floor covering. In particular, the device is equipped with one or more temperature sensors and humidity sensors placed underneath the floor covering, and is connected to a unit for wireless transmission of the measured temperature and humidity values to a receiver, outside the floor. The device is also equipped with a memory, in which information relevant to the floor covering is stored and can operate in wireless communication with the receiver. In case of a floor covering consisting of wooden elements, for which an underfloor heating system is located in a cement floor, the detection of the temperature between said floor covering elements and the cement floor is particularly relevant, to prevent damages to the wooden elements.

Although appreciated, however, this device is adapted to monitor temperature and humidity after laying the floor and not to provide information whether or not the screed on which the floor has to be layed is ready, i.e. it has appropriate humidity and temperature.

It is also known from DE 102012 208 050 a device for measuring temperature and/or humidity in the casting of cement or in the screed, i.e. after solidification of the casting, in particular for determining the residual moisture of screed. The measuring device has approximately the shape of a flat box and lies on top of a support layer. On the support layer, cement is casted to form a screed, on top of which has to be installed a floor covering, for instance a wooden covering. Inside the box of the measuring device, a measuring and communication circuit is arranged; the box is filled with a potting compound, and the measuring and communication circuit is potted with the potting compound and thus permanently integrated in the box and, with the box, in the screed.

The box includes a wall which is vapour permeable and in connection with the screed, so that a balance between the humidity in the box and the residual moisture of the screed sets. As a result, humidity in the box may be quite precise measured and communicated by means of the measuring and communication circuit to a remote reader, outside the creed.

This measuring device is appreciated for providing useful information as to whether or not the screed is ready to be covered by the floor covering, therefore preventing laying down the covering when the humidity or temperature is not within predetermined ranges and is also appreciated for preventing to waste time, since the covering may be laid down as soon as the temperature and humidity values are appropriate.

However, also this measuring device is affected by limitation that currently affects its usage.

First of all, the device is destined to remain forever in the screed, and with it all the electronic components integrated therein, including the measuring and communication circuit. This is not cost effective, considered that the electronic component are the ones more expensive.

As a consequence, to reduce costs, it happens that few measurement devices, in some cases only one device, is used to measure humidity and temperature of a casting of cement, also when the casting interests a wide area of several square meters.

On the other hand, being the measurement device completely embedded in the casting and subsequently in the screed, it is not possible to intervene on it in case of malfunctioning or in any other case, for instance when a supply battery is discharged. This may happen when time required for solidification and drying the cement is more than expected, also for environmental condition, and ultimately more than the duration of the battery. As a result, the humidity and temperature can no more be determined and the covering of the floor has to be made without precise understanding on the state of maturation of the screed. In this respect, it has also to be considered that control before laying (e.g.) parquet is always carried out after a CM test, according to SIA standard.

Furthermore, the screed is ultimately not homogenous in the area in correspondence of which the box is embedded, due to different material used to fill the box and material of the casting, for instance cement. This may lead to some minor problem, such as a different sound when walking on the covering over the box instead of on other covering of the floor.

The problem at the base of the present invention is that of providing a device for measuring the temperature and/or the humidity in a casting, and in the screed formed by solidification thereof, which is able to provide precise indication but at the same time less expensive, and therefore adapted to be widespread also in wide areas where the screed is formed, and adapted to be handled in case of malfunction or battery consumption, substantially overcoming all the limitations that currently affects the device for measuring the temperature and/or the humidity of the prior art. DE102011056548 and XP055710036 (Finland Oy Saint-Gobain: "P22 Accessories for flooring: Moisture sensor for floor screeds" are examples of sensor devices and methods for measuring the temperature or humidity of concrete.

### Summary of the invention

The idea at the base of the present invention is that of arranging electronic components for measuring the humidity and/or temperature of a casting of cement or the like, and therefore of a screed formed by the solidification thereof, in an space kept empty from the casting of cement, leaving, among said electronic components (preferably only) a sensor in contact with the casting.

Accordingly, (only) the sensor is definitely left inside the screed after solidification, therefore reducing the costs. Advantageously, a plurality of devices may be installed for a same casting, especially when a wide area has to be covered. Moreover, the electronic components may be handled in the space left empty from the casting, therefore allowing the possibility to intervene on them in case possible problems or to substitute a battery of the sensor. Moreover, when the screed is ready to be covered, the (small) space previously left empty, may be filled with same material of the casting, for instance cement. This may be done with a a fast curing, fast drying material such as Rapid Set Cement All.

According to the solution idea mentioned above the technical problem at the base of the present invention is solved by a method to measure temperature and/or humidity of cement, concrete or the like including
- arranging a sensor device on a floor before casting the cement or concrete on the floor, the sensor device including a casing, a circuit board inside the casing, and a temperature and/or humidity sensor connected to the circuit board and at least in part protruded outside the casing through a side wall thereof;
- casting, wherein portion of the sensor protruded outside the casing is covered by the casting;
- cutting the casing flush to the casting, in case protruding from the casting;
- measuring temperature and/or humidity; characterized by
- removing the circuit board from the casing after the cement or concrete has solidified, leaving the casing and the sensor in the cement or concrete;
- filling the casing with concrete, cement or other material.

Preferably, the method further includes fixing the casing at the floor before casting, for instance by means of one or more screws at a base of the casing or by means of an adhesive between the base and the floor.

The method also includes closing an aperture of the casing before casting for protecting the circuit board, preferably by applying a cover at the aperture, wherein the cover is removable from the casing.

In an embodiment, the step of measuring the temperature and/or humidity includes transmitting a value measured from the sensor to a remote device, wherein said transmission is made in radio communication, preferably via LORA protocol, by means of an antenna integrated or connected to the circuit board inside the casing, and wherein an operator is sent on place from a remote site to remove the circuit board from the casing, only if the value measured is above a predetermined threshold value of temperature and/or humidity.

In an embodiment, the step of measuring the temperature and/or humidity includes measuring at least one second value of temperature and/or humidity by means of at least one second sensor included in a corresponding at least one second sensor device and transmitting said at least one second value to the remote device, the at least one second sensor device being arranged at a different location in the casing with respect to the sensor device, wherein the operator is sent on place from the remote site to remove the circuit boards of the sensor device and of the at least one second sensor device, only if the temperature and/or humidity values measured from the sensor and from the at least one second sensor are above the predetermined threshold value of temperature and/or humidity.

Preferably, the remote device comprises means for storing and displaying a virtual map of the floor, wherein the sensor and the at least one second sensor are associated to corresponding identification numbers (IDs) and each of said identification number (ID) is associated to a corresponding location on the virtual map based on the corresponding identification numbers, and wherein different values of temperature or humidity measured by the sensor and by the at least one second sensor are displayed on corresponding locations on the virtual map.

For instance, the corresponding location on the virtual map is provided by means of a portable electronic device connected to the remote device, preferably via Internet, when the sensor device is arranged on the floor.

A sensor device outside the scope of the present invention for measuring a temperature and/or humidity of cement, concrete or the like is also disclosed, including:
- a casing,
- a circuit board inside the casing, and
- a temperature and/or humidity sensor connected to the circuit board and at least in part protruded outside the casing through a side wall thereof and arranged to be covered by a casting of cement or concrete;
wherein the circuit board is removable from the casing to fill the casing with concrete.

Preferably, the casing includes a base and means to fix the base to the floor, preferably screws or an adhesive.

The side wall of the casing is extended from a base of the casing and delimits an aperture of the casing, wherein the casing further includes a removable cover closing the aperture.

A plurality of side walls, preferably four side walls, are extended from the base and delimits an inner space of the casing, wherein a first portion of each of the side walls is fixed on the base, preferably in a perpendicular direction with respect to the base, and a second portion of each of the side wall is movable from the corresponding first portion, preferably hinged thereon, wherein said part of the casing is configured to be cut flush to the casing is the second portion of each of the side wall.

At least one of the second portions of the side wall may comprise an engagement profile adapted to engage at least a coupling profile of another one of said second portions of the side wall, wherein the engagement profile and coupling profile, engaged, prevent the at least one of second portions and the another one of the second portions from moving one with respect to the other and from the corresponding first portion.

The casing may include a rectangular base, four side walls perpendicular to the rectangular base, wherein two opposite side walls of the four side walls include engagement profiles and the other two opposed side walls include coupling profiles, and wherein the casing is parallelepiped when the engagement profiles are engaged to the coupling profiles.

The sensor protrudes from the first portion of one of the side walls, passing through an aperture of the first portion and closing the aperture, preferably hermetically.

The base may include means for snap engagement of the circuit board.

Further features and advantages of the method to measure a humidity and/or temperature of a casting of cement or the like according to the present invention will be apparent by an embodiment thereof, given here below with reference to the enclosed drawings, provided only with exemplificative and not limiting purpose.

### Brief description of drawings

Fig. 1 is a bottom-perspective view of a sensor device.
Fig. 2 is a top-perspective view of the sensor device of fig. 1.
Fig. 3 is a top view of the sensor device of fig. 1.
Fig. 4 is a cross side view of the sensor device of fig. 1.
Fig. 5 is the bottom-perspective view of the sensor device of fig. 1, wherefrom a sensor is removed.
Fig. 6 is a top-perspective view of the sensor device of fig. 5.
Fig. 7 is a top view of the sensor device of fig. 5.
Fig. 8 is another cross-side view of the sensor device of fig. 5.
Fig. 9 is a cross-side view of the sensor device of fig. 5.

### Detailed description of the invention

With reference to the annexed drawing, it is hereafter described a sensor device 1 for measuring a temperature and/or humidity of cement, concrete or the like.

The cement or concrete is a material in semi-solid form which is adapted to be laid down on a support and to mature in a screed of solid form and substantially flat surface, adapted to be covered by a floor covering, for instance a wooden floor, such as a parquet, or a floor of a porcelain stoneware tiles.

The sensor device 1 is used to measure the humidity and/or temperature of the casting cement, since it is important to wait for an appropriate state of maturation of the cement, corresponding to predetermined values of humidity and/or temperature, before installing the floor covering over the screed, in order to avoid damages to the covering, to guarantee the correct adhesion of glues or other concretes used to attach the covering to the screed and also to avoid that residual humidity is dispersed through the floor covering, after its application to the screed.

The sensor device 1 is adapted to be placed on the ground, for instance on a support or substrate previously prepared and adapted to receive the casting of cement thereon, and it is intended to enter in contact with the casting for measuring the humidity and/or temperature.

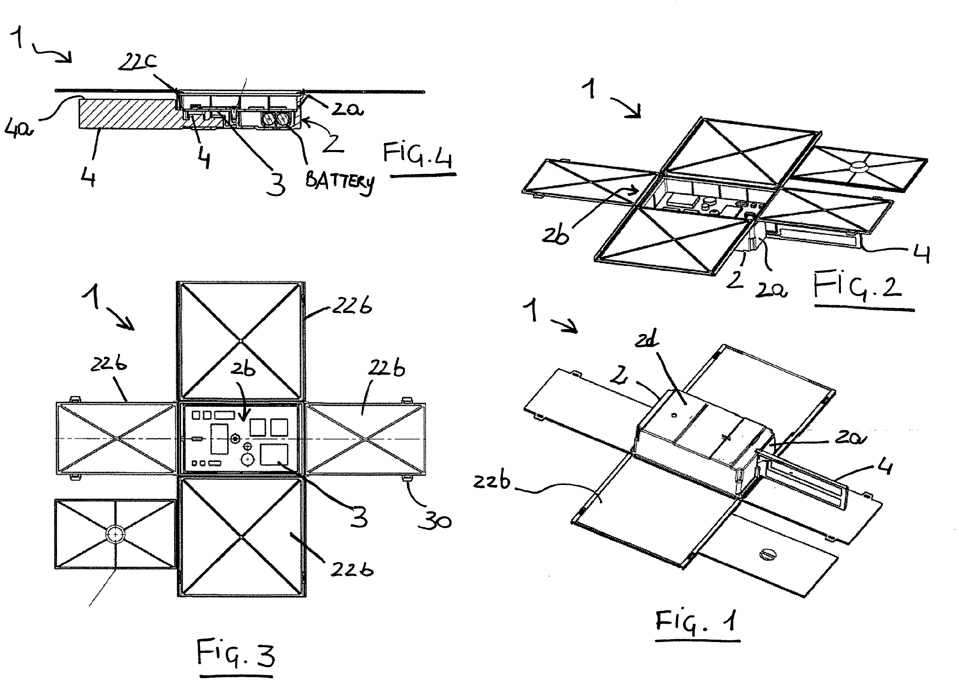

The sensor device 1 includes a casing 2, for instance a box like shape casing, a circuit board 3 inside the casing 2, and a temperature and/or humidity sensor 4 connected to the circuit board 3, as schematically represented in figure 1.

In particular, the casing 2 include sidewalls configured to enter in contact with the cement of the casting outside the casing 2, and the sensor 4 as well is configured to enter in contact with the cement outside the casing 2. A direct contact between a sensor probe of the sensor 4 and the cement allows to obtain more precise measurements.

In this respect, the sensor protrudes, at least in part, outside the casing 2, to directly contact the cement or concrete with the sensor probe, while the other electronic components of the circuit board 3, including an antenna and a battery, are protected inside the casing 2, and therefore not destined to contact the cement or concrete. The antenna in connected to a communication interface of the circuit board 3, and is programmed to transmit the values of temperatures and/or humidity measured by the sensor probe, to an external reader in radio communication with the communication interface. The battery supplies power to the electronic components of the circuit board 3.

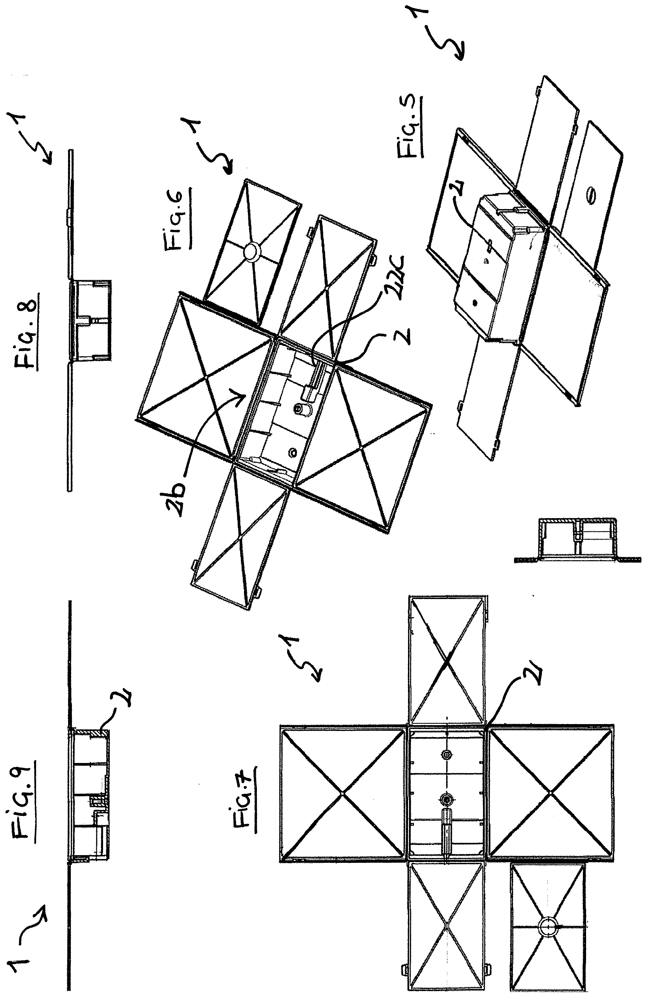

In the embodiment represented in figures 1-9, the sensor 4 is protruded outside the casing 2 through a side wall 2a of the casing. In particular, the side wall 2a may include an aperture through which the sensor 4 pass, in order to leave a portion of the sensor 4 (supporting the sensor probe) outside the casing 2 and another portion inside the casing 2, where a connection portion of the sensor 4 is plugged to the circuit board 3.

The connection portion of the sensor 4 is removable from the circuit board 3. In a preferred embodiment, the connection portion is configured to couple and de-couple to the circuit board by means of a movement of the circuit board 3, respectively, towards or backwards the bottom (base 2a) of the casing 2, in a direction substantially perpendicular to a surface of the bottom of the casing 2. Such a surface is flat, to adhere to the support where the casting is made.

For instance, the circuit board 3 may include a slot (i.e. connector port) and the spatial arrangement of the connection portion of the sensor 4 and of the slot of the circuit board 3 with respect to the casing 2 is such that physical insertion of the circuit board 3 in the casing 2 realizes also electrical connection of the sensor 4. In this respect, an aperture 2b of the casing 2, for instance a rectangular or circular aperture, arranged opposite to the bottom 2a of the casing 2, may correspond in shape and size, to the circuit board 3 and wall(s) of the aperture 2b guides the circuit board 3 toward the bottom of the casing 2, until the connection sensor 4-circuit board 3 is realized, preferably at a predetermined high with respect to the bottom 2a surface. Additionally, to improve the coupling / decoupling, guides and grooves may be provided in the wall(s) of the casing 2 and on edges of the circuit board 3.

A part of the casing 2 remaining outside the cement or concrete after casting is configured to be cut flush to the casting and to leave a remaining portion of the casing 2 in the solidified cement or concrete.

For instance, in case the casing 2 has a rectangular shape, four walls extending from the bottom 2a of the casing 2 are in contact with the cement up to a predetermined height, corresponding to an height of the screed to be formed, and the same walls have a portion protruding outside the casting, which is cut after casting, preferably after the casting has solidified at least in part.

The base 2a (also indicate above as bottom) of the casing 2 may be fixed to the ground or to the support whereon the casting has to be made, for instance by means of one or more screws or an adhesive substrate. The adhesive substrate may be integrated as a bi-adhesive strip having a first surface already attached to an outer surface of the base 2a and an opposite surface covered by a removable tape, which is removed leaving the opposite surface free, and ready to attach to the ground, when the casing has to be put in pose.

The aperture 2b of the casing 2 is delimited by sidewall 2a extended from the base 2a of the casing 2, and a removable cover 2c is further provided to close the aperture 2b. The aperture 2b is closed with the cover 2c to protect the electronic components therein during casting; the cover 2c is removed after casting to cut the sidewall 2a, therefore re-delimiting the aperture 2b at a lower height with respect to the base 2a; subsequently, the cover may be re-applied to the aperture 2b in order to protect the electronic components also after casting, during maturation of the cement. The cover 2c is finally removed so as the casing 2 may be filled with cement or other concrete, preferably the same material of the casting.

In a preferred embodiment, a plurality of side walls 2a, preferably four side walls 2a as represented in figures 1-9, are extended from the base 2a and delimits an inner space of the casing 2. A first portion 22a of each of the side walls 2a is fix on the base 2a of the casing, for instance in a perpendicular direction with respect to the base 2a, and a second portion 22b of each of the side wall 2a is movable from the corresponding first portion 22a, preferably hinged thereon. Advantageously, the second portions 22b may be distanced apart from the first portions 22a to facilitate access to the circuit board 3, which is arranged at the bottom side, surrounded by the first portions 22a. Before casting, the second portions 22b are reciprocated to the first portion, leaving only the aperture 2b of the casing open.

The part of the casing 2 configured to be cut flush to the casting of cement is the second portion 22b of each of the side wall 2a. Therefore, the aperture 2b on which the cover 2c is applied is the aperture delimited by the terminal end of the second portion 22, which is at a first distance from the base 2a before being cut and at a second distance, minor than, the first distance, after cut.

Preferably the terminal end of the second portion 22b are adapted to be engaged one to the other and are configured, when engaged, in a perpendicular direction with respect to the bottom surface of the casing 2. In such a configuration, each one of the second portions 22b is preferably flush with the corresponding first portions 22a of the casing.

In an embodiment, at least one of the second portions 22b of the side wall 2a comprises an engagement profile adapted to engage at least a coupling profile of another one of the second portions 22b of the side wall 2a. The engagement profile and coupling profile, when engaged, prevent the at least one of second portions and the another one of the second portions from moving one with respect to the other and from the corresponding first portion 22a, therefore avoiding that the sidewalls are deformed or moved by the casting, when the casting is made.

Still with reference to the not limiting embodiment of figure 1-9, the casing 2 includes a rectangular base 2a, four side walls 2a perpendicular to the rectangular base. Two opposite side walls include engagement profiles and the other two opposed side walls include coupling profiles. In this exemplary embodiment, the casing is parallelepiped when the engagement profiles are engaged to the coupling profiles. However, other shape of the casing and other arrangement of the engagement profiles are possible.

Here after are summarized the advantages of the present invention. Being all the electronic components inside the casing, except the sensor probe, protected from the casting, they may be reused, therefore reducing costs. To improve protection of electric components inside the casing, different measures may be adopted, for instance a gasket may be provide in the aperture of the sidewall through which the sensor pass, hermetically closing the aperture and preventing or reducing humidity flow towards the inner of the casing.

Moreover, the sensor probe being in direct contact with the casting, outside the casing, the measurement is more precise. Preferably, the sensor 4 protrudes from the first portion 22a of one of the sidewalls 2a, passing through the aperture 22c of the first portion 22a and closing the aperture 22c, hermetically.

Furthermore, the inner side of the casing 2 may be filled with same material of the casting, after removal of electronic components, leading to a more homogeneous composition of the screed.

## Claims

1. Method to measure temperature and/or humidity of cement, concrete or the like:
- arranging a sensor device (1) on a floor before casting the cement or concrete on the floor, the sensor device (1) including a casing (2), a circuit board (3) inside the casing (2), and a temperature and/or humidity sensor (4) connected to the circuit board (3) and at least in part protruded outside the casing (2) through a side wall (2a) thereof;
- casting, wherein portion (4a) of the sensor (4) protruded outside the casing (2) is covered by the casting;
- cutting the casing (2) flush to the casting, in case protruding from the casting;
- measuring temperature and/or humidity; **characterized by**
- removing the circuit board (3) from the casing (2) after the cement or concrete has solidified, leaving the casing (2) and the sensor (4) in the cement or concrete;
- filling the casing (2) with concrete, cement or other material.

2. Method according to claim 1 including: fixing the casing (2) at the floor before casting, preferably by means of one or more screws at a base (2d) of the casing (2) or by means of an adhesive between the base (2d) and the floor.

3. Method according to claim 1 including: closing an aperture (2b) of the casing (2) before casting for protecting the circuit board (3), preferably by applying a cover (2c) at the aperture (2b), wherein the cover (2c) is removable from the casing (2).

4. Method according to claim 1 wherein said step of measuring the temperature and/or humidity includes transmitting a value measured from the sensor (4) to a remote device, wherein said transmission is made in radio communication, preferably via LORA protocol, by means of an antenna integrated or connected to the circuit board (3) inside the casing (2), and wherein an operator is sent on place from a remote site to remove the circuit board (3) from the casing, only if the value measured is above a predetermined threshold value of temperature and/or humidity.

5. Method according to claim 4 wherein said step of measuring the temperature and/or humidity includes measuring at least one second value of temperature and/or humidity by means of at least one second sensor (4) included in a corresponding at least one second sensor device (1) and transmitting said at least one second value to the remote device, said at least one second sensor device (1) being arranged at a different location in the casing with respect to the sensor device (1), wherein the operator is sent on place from the remote site to remove the circuit boards (3) of the sensor device (1) and of the at least one second sensor device (1), only if the temperature and/or humidity values measured from the sensor (4) and from the at least one second sensor (4) are above the predetermined threshold value of temperature and/or humidity.

6. Method according to claims 4 and 5 wherein said remote device comprises means for storing and displaying a virtual map of the floor, wherein the sensor (4) and the at least one second sensor (4) are associated to corresponding identification numbers (IDs) and each of said identification number (ID) is associated to a corresponding location on the virtual map based on the corresponding identification numbers (IDs), and wherein different values of temperature or humidity measured by the sensor (4) and by the at least one second sensor (4) are displayed on corresponding locations on the virtual map.

7. Method according to claim 6 wherein said corresponding location on the virtual map is provided by means of a portable electronic device connected to the remote device, preferably via Internet, when the sensor device (1) is arranged on the floor.

## Patentansprüche

1. Verfahren zur Messung der Temperatur und/oder Feuchtigkeit von Zement, Beton oder dergleichen, umfassend:
Anordnen einer Sensoreinrichtung (1) auf einem Boden vor dem Gießen des Zements oder Betons auf den Boden, wobei die Sensoreinrichtung (1) ein Gehäuse (2), eine Leiterplatte (3) innerhalb des Gehäuses (2) sowie einen Temperatur- und/oder Feuchtigkeitssensor (4) umfasst, der mit der Leiterplatte (3) verbunden ist und zumindest teilweise durch eine Seitenwand (2a) des Gehäuses (2) nach außen ragt;
Gießen, wobei ein Abschnitt (4a) des Sensors (4), der außerhalb des Gehäuses (2) hervorsteht, durch das Gießmaterial bedeckt wird;
Abschneiden des Gehäuses (2) bündig mit dem Gießmaterial, falls es aus dem Gießmaterial hervorsteht;
Messen der Temperatur und/oder Feuchtigkeit;
**dadurch gekennzeichnet, dass**
die Leiterplatte (3) nach dem Erstarren des Zements oder Betons aus dem Gehäuse (2) entfernt wird, während das Gehäuse (2) und der Sensor (4) im Zement oder Beton verbleiben;
das Gehäuse (2) mit Beton, Zement oder einem anderen Material gefüllt wird.

2. Verfahren nach Anspruch 1, umfassend:
Befestigen des Gehäuses (2) am Boden vor dem Gießen, vorzugsweise mittels einer oder mehrerer Schrauben an einer Basis (2d) des Gehäuses (2) oder mittels eines Klebstoffs zwischen der Basis (2d) und dem Boden.

3. Verfahren nach Anspruch 1, umfassend:
Verschließen einer Öffnung (2b) des Gehäuses (2) vor dem Gießen zum Schutz der Leiterplatte (3), vorzugsweise durch Anbringen einer Abdeckung (2c) an der Öffnung (2b), wobei die Abdeckung (2c) vom Gehäuse (2) entfernbar ist.

4. Verfahren nach Anspruch 1, wobei der Schritt des Messens der Temperatur und/oder Feuchtigkeit umfasst Übertragen eines vom Sensor (4) gemessenen Werts an ein entferntes Gerät, wobei die Übertragung drahtlos erfolgt, vorzugsweise über das LORA-Protokoll, mittels einer in die Leiterplatte (3) integrierten oder mit ihr verbundenen Antenne, und wobei ein Bediener von einem entfernten Standort aus zum Ort geschickt wird, um die Leiterplatte (3) aus dem Gehäuse (2) zu entfernen, nur wenn der gemessene Wert über einem vorgegebenen Schwellenwert für Temperatur und/oder Feuchtigkeit liegt.

5. Verfahren nach Anspruch 4, wobei der Schritt des Messens der Temperatur und/oder Feuchtigkeit umfasst Messen mindestens eines zweiten Temperatur- und/oder Feuchtigkeitswerts mittels mindestens eines zweiten Sensors (4), der in einer entsprechenden mindestens einen zweiten Sensoreinrichtung (1) enthalten ist, und Übertragen des mindestens einen zweiten Werts an das entfernte Gerät, wobei die mindestens eine zweite Sensoreinrichtung (1) an einer anderen Stelle im Gehäuse als die erste Sensoreinrichtung (1) angeordnet ist, und wobei der Bediener von einem entfernten Standort aus zum Ort geschickt wird, um die Leiterplatten (3) der Sensoreinrichtung (1) und der mindestens einen zweiten Sensoreinrichtung (1) zu entfernen, nur wenn die von Sensor (4) und mindestens einem zweiten Sensor (4) gemessenen Temperatur- und/oder Feuchtigkeitswerte über dem vorgegebenen Schwellenwert liegen.

6. Verfahren nach den Ansprüchen 4 und 5, wobei das entfernte Gerät Mittel zum Speichern und Anzeigen einer virtuellen Karte des Bodens umfasst, wobei der Sensor (4) und der mindestens eine zweite Sensor (4) entsprechenden Identifikationsnummern (IDs) zugeordnet sind, und wobei jede dieser Identifikationsnummern (ID) einem entsprechenden Ort auf der virtuellen Karte basierend auf den zugehörigen Identifikationsnummern (IDs) zugeordnet ist, und wobei unterschiedliche von Sensor (4) und mindestens einem zweiten Sensor (4) gemessene Temperatur- oder Feuchtigkeitswerte an den entsprechenden Orten auf der virtuellen Karte angezeigt werden.

7. Verfahren nach Anspruch 6, wobei der entsprechende Ort auf der virtuellen Karte durch ein tragbares elektronisches Gerät bereitgestellt wird, das vorzugsweise über das Internet mit dem entfernten Gerät verbunden ist, wenn die Sensoreinrichtung (1) auf dem Boden angeordnet wird.

## Revendications

1. Procédé de mesure de la température et/ou de l'humidité du ciment, du béton ou d'un matériau similaire, comprenant :
- la disposition d'un dispositif de capteur (1) sur un sol avant le coulage du ciment ou du béton sur le sol, ledit dispositif de capteur (1) comprenant un boîtier (2), une carte de circuit imprimé (3) à l'intérieur du boîtier (2), et un capteur de température et/ou d'humidité (4) connecté à la carte de circuit imprimé (3) et faisant saillie au moins en partie à l'extérieur du boîtier (2) à travers une paroi latérale (2a) de celui-ci;
- le coulage, dans lequel une partie (4a) du capteur (4) qui fait saillie à l'extérieur du boîtier (2) soit recouverte par le matériau coulé;
- la coupe du boîtier (2) à ras du coulage, dans le cas où il dépasse du coulage;
- la mesure de la température et/ou l'humidité; **caractérisé par**
- le retrait de la carte de circuit imprimé (3) du boîtier (2) après la solidification du ciment ou du béton, en laissant le boîtier (2) et le capteur (4) dans le ciment ou le béton ;
- le remplissage du boîtier (2) avec du béton, du ciment ou un autre matériau.

2. Procédé selon la revendication 1, comprenant : la fixation du boîtier (2) sur le sol avant le coulage, de préférence au moyen d'une ou de plusieurs vis disposées sur une base (2d) du boîtier (2), ou par l'intermédiaire d'un adhésif entre la base (2d) et le sol.

3. Procédé selon la revendication 1, comprenant : la fermeture d'une ouverture (2b) du boîtier (2) avant le coulage afin de protéger la carte de circuit imprimé (3), de préférence en posant un couvercle (2c) sur l'ouverture (2b), dans lequel ledit couvercle (2c) est amovible du boîtier (2).

4. Procédé selon la revendication 1, dans lequel ladite étape de mesure de la température et/ou de l'humidité comprend la transmission d'une valeur mesurée par le capteur (4) à un dispositif distant, dans lequel ladite transmission est effectuée par communication radio, de préférence via le protocole LORA, au moyen d'une antenne intégrée ou connectée à la carte de circuit imprimé (3) à l'intérieur du boîtier (2), et dans lequel un opérateur est envoyé sur place à partir d'un site distant pour retirer la carte de circuit imprimé (3) du boîtier, uniquement si la valeur mesurée est supérieure à une valeur seuil prédéterminée de température et/ou d'humidité.

5. Procédé selon la revendication 4, dans lequel ladite étape de mesure de la température et/ou de l'humidité comprend la mesure d'au moins une seconde valeur de température et/ou d'humidité au moyen d'au moins un second capteur (4) compris dans l'au moins un second dispositif de capteur (1) correspondant et la transmission de ladite au moins seconde valeur au dispositif distant, l'au moins un second dispositif de capteur (1) étant disposé à un emplacement distinct dans le boîtier par rapport au premier dispositif de capteur (1), dans lequel l'opérateur est envoyé sur place depuis le site distant pour retirer les cartes de circuit imprimé (3) du dispositif de capteur (1) et de l'au moins un second dispositif de capteur (1), uniquement si les valeurs de température et/ou d'humidité mesurées par le capteur (4) et par l'au moins un second capteur (4) sont supérieures à la valeur seuil prédéterminée de température et/ou d'humidité.

6. Procédé selon les revendications 4 et 5, dans lequel ledit dispositif distant comprend des moyens pour stocker et afficher une carte virtuelle du sol, dans lequel le capteur (4) et l'au moins un second capteur (4) sont associés à des numéros d'identification (IDs) respectifs, et chacun desdits numéros d'identification (ID) étant associé à un emplacement correspondant sur la carte virtuelle en fonction desdits numéros d'identification (IDs), et dans lequel les différentes valeurs de température ou d'humidité mesurées par le capteur (4) et par l'au moins un capteur (4) sont affichées aux emplacements correspondants sur la carte virtuelle.

7. Procédé selon la revendication 6, dans lequel ledit emplacement correspondant sur la carte virtuelle est fourni par l'intermédiaire d'un dispositif électronique portable connecté au dispositif distant, de préférence grâce à Internet, lorsque le dispositif de capteur (1) est disposé sur le sol.
